Europäisches Patentamt

European Patent Office

Office européen des brevets

(19)

(11) **EP 0 934 746 B1**

(12) **EUROPEAN PATENT SPECIFICATION**

(45) Date of publication and mention
of the grant of the patent:
**22.10.2003 Bulletin 2003/43**

(51) Int Cl.[7]: **A61K 35/78**, A61K 9/08,
A61P 35/00

(21) Application number: **97949286.5**

(22) Date of filing: **03.12.1997**

(86) International application number:
**PCT/RU97/00390**

(87) International publication number:
**WO 98/024458 (11.06.1998 Gazette 1998/23)**

(54) **SUBSTANCE IN THE FORM OF AN AQUEOUS EXTRACT OF VEGETAL RAW MATERIAL FOR TREATING ONCOLOGICAL DISEASES, AND METHOD FOR PRODUCING THE SAME**

SUBSTANZ IN FORM EINES WÄSSRIGEN EXTRAKTS VON PFLANZENROHMATERIAL ZUR BEHANDLUNG ONKOLOGISCHER KRANKHEITEN SOWIE VERFAHREN ZU DEREN HERSTELLUNG

SUBSTANCE PERMETTANT DE TRAITER DES AFFECTIONS ONCOLOGIQUES ET CONSISTANT EN DES EXTRAITS AQUEUX DE MATIERES PREMIERES NATURELLES, ET PROCEDE DE PRODUCTION DE CETTE SUBSTANCE

(84) Designated Contracting States:
**AT CH DE FR IT LI NL**

(30) Priority: **04.12.1996 RU 96123079**

(43) Date of publication of application:
**11.08.1999 Bulletin 1999/32**

(73) Proprietors:
• **Gosudarstvenny Nauchny Tsentr Rossiiskoi Federatsii "NIOPIK" (GNTS RF "NIOPIK")**
**Moscow, 103787 (RU)**
• **Novosibirsky Institut Organicheskoi Khimii**
**Novosibirsk, 630090 (RU)**
• **MOSKOVSKY NAUCHNO-ISSLEDOVATELSKY ONKOLOGICHESKY**
**INSTITUT IMENI P.A. GERTSENA**
**Moscow, 125284 (RU)**

(72) Inventors:
• **VOROZHTSOV, Georgy Nikolaevich**
**Moscow, 107078 (RU)**
• **VINOKUROVA, Elena Jurievna**
**Novosibirsk, 630090 (RU)**
• **GAVRILJUK, Olga Alexeevna**
**Novosibirsk, 630090 (RU)**

• **KAZACHKINA, Natalya Ivanovna**
**Moskovskaya obl., Dzerzhinsk, 140051 (RU)**
• **LUZHKOV, Jury Mikhailovich**
**Moscow, 103032 (RU)**
• **NEMTSOVA, Elena Romanovna**
**Moscow, 123448 (RU)**
• **TOLSTIKOV, Genrikh Alexandrovich**
**Novosibirsk, 630090 (RU)**
• **CHISSOV, Valery Ivanovich**
**Moscow, 123364 (RU)**
• **SHULTS, Elvira Eduardovna**
**Novosibirsk, 630117 (RU)**
• **YAKUBOVSKAYA, Raisa Ivanovna**
**Moscow, 119270 (RU)**

(74) Representative: **Cortey, Pierre et al**
**Cabinet Hirsch**
**34, rue de Bassano**
**75008 Paris (FR)**

(56) References cited:
**FR-A- 2 502 957**          **FR-A- 2 532 179**
**FR-A- 2 733 419**          **GB-A- 2 226 494**
**RU-C- 2 045 271**          **RU-C- 2 080 867**
**US-A- 4 405 613**

**Description**

The present invention relates to medicine, particularly to phytotherapy, and still more particularly to a composition for treating oncological diseases, said composition comprising an aqueous extract of vegetable stock, and to a process for preparing thereof.

An anticancer preparation is known in the art, comprising as one of components an extract of a plant of the genus Geranium. The formulation of this preparation comprises also sulfuric acid, boric acid, an alcohol, and glycerol [1].

A disadvantage of this composition is that it can be recommended for external application only.

A composition is known, recommended as an auxiliary means which enhances the antitumoric activity, whose formulation, among other aqueous or aqueous-organic extracts of plants, comprises extracts of Astragalis, Pasonia, and Angelica [2].

Also known in the art is a multicomponent composition for antitumoric use, comprising plants of the genera Plantago and Angelica as components [3].

These compositions are disadvantageous in being multicomponent.

The present invention was directed to the provision of a composition for treating oncological diseases in the form of aqueous extracts of vegetable stock, said stock featuring a sufficiently simple composition, and to the provision of a process for preparing thereof.

For solving the posed problem, a composition is proposed, comprising aqueous extracts of plants of the genera Geranium, Plantago, plant of the genus Calendula officinalis, prepared with the following ratio of water and plants, g:

| Geranium | 300-360 fresh or 10-60 dry |
| Plantago | 10-60 dry |
| Calendula officinalis | 10-60 dry |
| Water | 3000 |

and Pentaphylloides fruticosa in an amount of 10-60 g (dry). Besides Pentaphylloides fruticosa, a plant of the genus Angelica in an amount of 10-60 g (dry) can be added. The use of separate components of the proposed composition in amounts smaller than the lower levels of said ranges lowers the therapeutic effect, whereas the upper levels of said ranges are limited by the volume of water, required for preparing said aqueous extracts.

In the proposed composition the plant of the genus Geranium can be represented by the species sibiricum, pratense, and robertianum; Plantago can be represented by the species lanceolata and media; Angelica can be represented by the species sylvestris and decurrens.

A process is known for preparing an extract of plants by treating them with water or with an aqueous-organic solvent [4].

A process of preparing a composition of vegetable origin is also known, which comprises extraction of comminuted grass with water by maceration, followed by filtration and re-maceration of the vegetable stock [5]. This process appears to be technologically complicated, since it involves a two-stage maceration.

It is an object of the proposed invention to provide a simple process for preparing an aqueous extract of plants for treating oncological diseases. To solve this problem, it is proposed to macerate plants (grass, leaves or roots) in water, with subsequent straining and centrifugation.

The biological activity of the plant extracts was assayed by determining the antioxidant and cytotoxic activity in vitro, as well as the toxicity and antitumoric activity in vivo.

The antioxidant activity of the extracts was determined from the ability of a sample to inhibit the lipid peroxidation processes (LPP) in murine liver homogenate [6].

The cytotoxic activity of the extracts in vitro was determined with the help of a biological test based on inhibition of the proliferation of the inoculated cell culture of human pulmonary adenocarcinoma A-549 [7].

The antitumoric activity of the extracts was assessed on mice $BDF_1$ suffering from leukemia, using different doses of the extracts. The assessment criteria were inhibition of the tumor growth and an increase in the lifetime of the animals.

In addition, the effect of the extracts on the therapeutic activity of the cytostatic cis-dichlorodiamine platinum (DDP) [8] was estimated.

The proposed invention is illustrated by the Examples presented hereinbelow.

Comparative Example 1

360 g of comminuted fresh grass Geranium sibiricum, 25 g of dry leaves of Plantago lanceolata, and 25 g of dry leaves of Calendula officinalis are soaked in 3000 g of water at 25°C for 8 days. On completion of the maceration procedure, the extract is strained and centrifugated under standard conditions (20°C, 3000 rpm, 40 min). The precipitate is discarded, and the extract is used.

Comparative Examples 1a-1e

Extracts of plants are prepared by following the procedure of Example 1, but the quantity of the mixture components and the maceration conditions are varied. The formulations are specified in Table 1.

Examples 2-2h

Extracts of plants are prepared by following the procedure of Example 1, but comminuted dry sprouts of the plant Pentaphylloides fruticosa are added to the formulation of the mixture being soaked. The formulations of the vegetable stock and the maceration conditions are specified in Table 1.

Examples 3-3f

Extracts of plants are prepared by following the procedure of Example 1, but, in addition to Pentaphylloides fruticosa, comminuted underground organs of the plant Angelica (species sylvestris and decurrens) are introduced into the formulation of the mixture being soaked. The formulations of the vegetable stock and the maceration conditions are specified in Table 1.

Table 1

| Ex. No. | Description of Stock | Consistence of stock | Weight (g) | Temp. (°C) | Time (days) |
|---------|----------------------|----------------------|------------|------------|-------------|
| 1 | Grass Geranium sibiricum | Fresh | 360 | 28 | 8 |
| | Leaf of Calendula officinalis | Dry | 25 | | |
| | Leaf of Plantago lanceolata | Dry | 25 | | |
| 1a | Grass Geranium pratense | Dry | 60 | 26 | 7 |
| | Leaf of Calendula officinalis | Dry | 10 | | |
| | Leaf of Plantago lanceolata | Dry | 10 | | |
| 1b | Grass Geranium pratense | Dry | 25 | 25 | 8 |
| | Leaf of Calendula officinalis | Dry | 25 | | |
| | Leaf of Plantago lanceolata | Dry | 25 | | |
| 1c | Grass Geranium sibiricum | Fresh | 300 | 28 | 7 |
| | Leaf of Calendula officinalis | Dry | 60 | | |
| | Leaf of Plantago lanceoloata | Dry | 10 | | |
| 1d | Grass Geranium sibiricum | Fresh | 320 | 27 | 6 |
| | Leaf of Calendula officinalis | Dry | 10 | | |
| | Leaf of Plantago lanceoloata | Dry | 60 | | |
| 1e | Grass Geranium sibiricum | Dry | 10 | 28 | 7 |
| | Leaf of Calendula officinalis | Dry | 60 | | |
| | Leaf of Plantago media | | 25 | | |
| 2 | Grass Geranium sibiricum | Fresh | 360 | 26 | 8 |
| | Leaf of Calendula officinalis | Dry | 25 | | |
| | Leaf of Plantago lanceolata | Dry | 25 | | |

Table 1   (continued)

| Ex. No. | Description of Stock | Consistence of stock | Weight (g) | Temp. (°C) | Time (days) |
|---------|---------------------|----------------------|------------|------------|-------------|
| 2a | Sprouts of Pentaphylloides fruticosa | Dry | 25 | | |
| | Grass Geranium sibiricum | Fresh | 360 | 25 | 6 |
| | Leaf of Calendula officinalis | Dry | 25 | | |
| | Leaf of Plantago lanceolata | Dry | 25 | | |
| | Sprouts of Pentaphylloides fruticosa | Dry | 10 | | |
| 2b | Grass Geranium sibiricum | Fresh | 360 | 25 | 6 |
| | Leaf of Calendula officinalis | Dry | 25 | | |
| | Leaf of Plantago lanceolata | Dry | 25 | | |
| | Sprouts of Pentaphylloides fruticosa | Dry | 10 | | |
| 2c | Grass Geranium robertianum | Dry | 60 | 28 | 7 |
| | Leaf of Calendula officinalis | Dry | 25 | | |
| | Leaf of Plantago lanceolata | Dry | 25 | | |
| | Sprouts of Pentaphylloides fruticosa | Dry | 10 | | |
| 2d | Grass Geranium robertianum | Dry | 10 | 28 | 7 |
| | Leaf of Calendula officinalis | Dry | 25 | | |
| | Leaf of Plantago lanceolata | Dry | 25 | | |
| | Sprouts of Pentaphylloides fruticosa | Dry | 60 | | |
| 2e | Grass Geranium sibiricum | Fresh | 360 | 28 | 8 |
| | Leaf of Calendula officinalis | Dry | 10 | | |
| | Leaf of Plantago lanceolata | Dry | 10 | | |
| | Sprouts of Pentaphylloides fruticosa | Dry | 10 | | |
| 2f | Grass Geranium sibiricum | Fresh | 360 | 28 | 8 |
| | Leaf of Calendula officinalis | Dry | 60 | | |
| | Leaf of Plantago lanceolata | Dry | 10 | | |
| | Sprouts of Pentaphylloides fruticosa | Dry | 10 | | |
| 2h | Grass Geranium sibiricum | Fresh | 360 | 28 | 8 |
| | Leaf of Calendula officinalis | Dry | 10 | | |
| | Leaf of Plantago lanceolata | Dry | 60 | | |
| | Sprouts of Pentaphylloides fruticosa | Dry | 10 | | |
| 3 | Grass Geranium pratense | Dry | 10 | 28 | 6 |
| | Leaf of Calendula officinalis | Dry | 25 | | |
| | Leaf of Plantago media | Dry | 25 | | |
| | Sprouts of Pentaphylloides fruticosa | Dry | 25 | | |
| | Root of Angelica silvestris | Dry | 60 | | |

Table 1   (continued)

| Ex. No. | Description of Stock | Consistence of stock | Weight (g) | Temp. (°C) | Time (days) |
|---------|---------------------|---------------------|-----------|-----------|-------------|
| 3a | Grass Geranium pratense | Dry | 10 | 28 | 6 |
|    | Leaf of Calendula officinalis | Dry | 25 | | |
|    | Leaf of Plantago media | Dry | 25 | | |
|    | Sprouts of Pentaphylloides fruticosa | Dry | 10 | | |
|    | Root of Angelica silvestris | Dry | 60 | | |
| 3b | Grass Geranium pratense | Dry | 60 | 27 | 7 |
|    | Leaf of Calendula officinalis | Dry | 25 | | |
|    | Leaf of Plantago media | Dry | 25 | | |
|    | Sprouts of Pentaphylloides fruticosa | Dry | 10 | | |
|    | Root of Angelica decurrens | Dry | 10 | | |
| 3c | Grass Geranium pratense | Dry | 40 | 27 | 7 |
|    | Leaf of Calendula officinalis | Dry | 25 | | |
|    | Leaf of Plantago media | Dry | 25 | | |
|    | Sprouts of Pentaphylloides fruticosa | Dry | 10 | | |
|    | Root of Angelica decurrens | Dry | 10 | | |
| 3d | Grass Geranium pratense | Dry | 60 | 27 | 8 |
|    | Leaf of Calendula officinalis | Dry | 25 | | |
|    | Leaf of Plantago lanceolata | Dry | 25 | | |
|    | Sprouts of Pentaphylloides fruticosa | Dry | 25 | | |
|    | Root of Angelica decurrens | Dry | 60 | | |
| 3e | Grass Geranium pratense | Dry | 60 | 28 | 8 |
|    | Leaf of Calendula officinalis | Dry | 25 | | |
|    | Leaf of Plantago lanceolata | Dry | 25 | | |
|    | Sprouts of Pentaphylloides fruticosa | Dry | 40 | | |
|    | Root of Angelica silvestris | Dry | 30 | | |
| 3f | Grass Geranium pratense | Dry | 60 | 26 | 7 |
|    | Leaf of Calendula officinalis | Dry | 10 | | |
|    | Leaf of Plantago lanceolata | Dry | 10 | | |
|    | Sprouts of Pentaphylloides fruticosa | Dry | 10 | | |
|    | Root of Angelica silvestris | Dry | 60 | | |

Determination of the Antioxidant Activity of vegetable Extracts

The antioxidant activity was used by using a method based on the ability of the sample being investigated to inhibit LPP processes in murine liver homogenate [6]. The liver of BDF-1 (nondescript) mice was homogenized in a glass homogenizer and then centrifugated. The liver homogenate was introduced in an amount of 5-6 mg of protein per assay. The volume of the assay was brought to 0.5 ml with a solution of the sample being tested for the antioxidant activity. The assays were incubated for 3 h at 37°C.

The concentration of the LPP products was determined from the color reaction with thiobarbituric acid. The quantity of the antioxidant required for the 50% inhibition of LPP was adopted as a conventional unit of the sample under investigation.

The results are presented in Tables 2 and 2a.

Table 2

| Comparison of the antioxidant activity of samples of vegetable extracts with the activity of prior art antioxidant preparations | | |
|---|---|---|
| Nos. | Preparation | Antioxidant activity, conv. units (1/mg) |
| 1 | 1 | 333 |
| 2 | 2 | 333 |
| 3 | 3 | 333 |
| 4 | Retinol acetate (vitamin A) | 83 |
| 5 | Ascorbic acid (vitamin C) | 12 |
| 6 | β-Carotene | 6 |

Table 2a

| Comparison of the antioxidant activity of samples of vegetable extracts under storage | | | |
|---|---|---|---|
| Ex. No. | Quantity of sample, required for attaining 50% inhibition of LPP, mg/ml of assay | | |
| | 2 days after preparation | 16 days after preparation | 30 days after preparation |
| 1 | $3.0 \times 10^{-3}$ | $9.0 \times 10^{-3}$ | $9.2 \times 10^3$ |
| 1b | $7 \times 10^{-3}$ | $1.2 \times 10^{-2}$ | $1.3 \times 10^{-2}$ |
| 1c | $3.2 \times 10^{-3}$ | $9.1 \times 10^{-3}$ | $9.0 \times 10^3$ |
| 2 | $3.4 \times 10^{-3}$ | $7.0 \times 10^{-3}$ | $6.9 \times 10^3$ |
| 2d | $6.8 \times 10^{-3}$ | $6.9 \times 10^{-3}$ | $7.0 \times 10^3$ |
| 2h | $3.8 \times 10^{-3}$ | $6.9 \times 10^{-3}$ | $6.8 \times 10^3$ |
| 3 | $3.8 \times 10^{-3}$ | $6.0 \times 10^{-3}$ | $6.3 \times 10^3$ |
| 3b | $7.1 \times 10^{-3}$ | $1.5 \times 10^{-2}$ | $1.5 \times 10^{-2}$ |
| 3c | $9.2 \times 10^{-3}$ | $1.9 \times 10^{-2}$ | $2.0 \times 10^{-2}$ |

As can be seen from Table 2, extracts 1, 2, and 3 have a high antioxidant activity in the test in vitro, which exceeds appreciably the antioxidant activity of the known antioxidative vitamins: A, C and β-carotene (4.28 and 55-fold, respectively). On storage of the vegetable extracts (Table 2a) for 16-30 days, their antioxidant activity becomes lower by a factor of 1.5-2 due to the formation of a precipitate, into which active components are involved as well. Nevertheless, the antioxidant activity remains appreciably higher than the activity of vitamin preparations.

Determination of the Cytotoxic Activity of Extract Samples in vitro

When carrying out the cytotoxic biological test in vitro, each well of a flat-bottomed 96-well microtiter (Sarstedt, USA) was charged with 10 μl of a suspension of A549-line cells in a concentration of $1 \times 10^5$ cell/ml; 24 h later series dilutions of the vegetable preparations in the range of 1:4-1:64 were added, this corresponding to 3.0-0.1 mg/ml in the volume of 100 μl/well. The cytotoxic activity of the extracts and DDP was estimated colorimetrically [7]. The results were expressed in the percentage of inhibition of the A-549 test-culture proliferation. The cytotoxic activity of the sample was adopted to be the maximum titer of the sample, at which the biologically significant (50%) level of inhibition of the A-549 test-culture proliferation was observed.

The results are presented in Table 3.

Table 3

| Cytotoxic activity of samples of vegetable extracts in terms of inhibition of the proliferation of cell culture of human pulmonary adenocarcinoma A-549 | | |
|---|---|---|
| Example No. | Maximum titer | Inhibition, % |
| 1 | 1/8 (1.2 mg/ml) | 50 |
| 1a | 1/8 (1.2 mg/ml) | 58 |
| 1b | 1/8 (1.2 mg/ml) | 60 |
| 1c | 1/8 (1.2 mg/ml) | 59 |
| 2 | 1/64 (0.1 mg/ml) | 66 |
| 2a | 1/64 (0.1 mg/ml) | 80 |
| 2b | 1/64 (0.1 mg/ml) | 72 |
| 2d | 1/64 (0.1 mg/ml) | 78 |
| 3 | 1/4 (3.0 mg/ml) | 79 |
| 3a | 1/4 (3.0 mg/ml) | 83 |
| 3c | 1/4 (3.0 mg/ml) | 80 |
| DDP | 10 μg/ml | 68 |

As can be seen from Table 3, the investigated samples of the vegetable extracts have a cytotoxic activity in the experiments in vitro. The maximum ability to inhibit proliferation of the cell culture A-549 was observed in sample 2 (66-80% at the concentration of 0.1 mg/ml), but it was one tenth that of DDP (68% at the concentration of 0.01 mg/ml), i.e., of the preparation conventionally used for induced cytotoxicity control.

Investigation of Tolerance to Vegetable Extract Samples

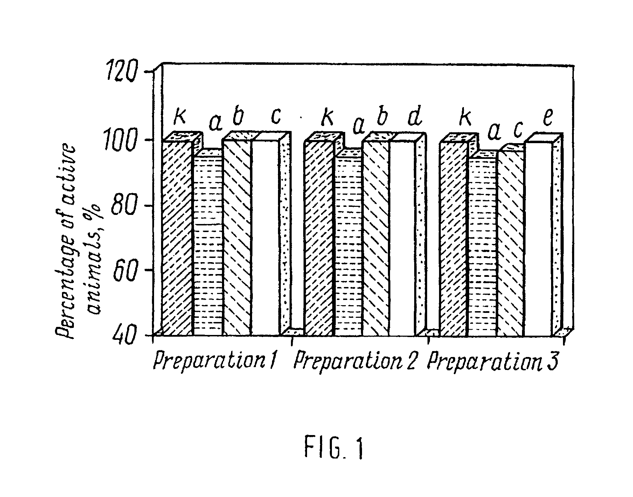

The tolerance to and harmlessness of the samples were investigated on animals with L-1210 inoculated subcutaneously. Samples of the extracts were administered through a probe many times throughout the lifetime of the animals, starting with the first day of the tumor growth, in single doses of 5.4 and 10.8 ml/kg in the volume of 0.2 ml or intraperitoneally in the volume of 1 ml. Control mice were administered a 0.9% NaCl solution instead of the vegetable extracts.
The toxic effect was estimated from the change in the body weight of mice and also from their external appearance (behavior, the appearance of the hair-cover, their motor activity). For a quantitative estimation of changes in the motor activity, an integral characteristic was introduced: $(a/b) \times 100\%$, where a is the total number of mice with a lowered motor activity from the 1st to the 6th day of the tumor growth, b is the total number of observations from the 1st to the 6th day of the tumor growth (1 observation - 1 living mouse per day).
The results of the experiments showed that the vegetable extracts in the single dose of 5.4 ml/kg do not affect the body weight, behavior, the external appearance of the hair-cover or the mobility of the mice. The administration of the vegetable extracts of some of the series in the single dose of 10.8 ml/kg brought about a weak toxic effect manifested in a reduction of the motor activity of the test animals in 5-10% of the observations (Fig. 1).
An increase of the single dose of the vegetable extracts to 1 ml (maximum permissible volume) in the case of single intraperitoneal administration of the preparations of series b, c, d did not lead to the development of visible disturbances in the behavior and external appearance of the animals or cause their death. This suggests that the $LD_{10}$, $LD_{50}$, and $LD_{100}$ values cannot be determined for these particular vegetable extracts.

Determination of the Antitumoric Activity of Vegetable Extract Samples

Lymphoid leukemia L1210 was inoculated to $BDF_1$ males in a sterile physiologic salt solution, $3 \times 10^6$ cells in 0.2 ml volume. The day of the inoculation was regarded as the zero day of tumor growth. The extract samples and DDP were administered in the doses and under the conditions specified in Tables 4, 5.
Treatment of the animals was started 24 hours after the tumor inoculation, control animals were administered a physiologic salt solution instead of the vegetable extracts. The antitumoric effect was estimated from the tumor growth inhibition (TGI) calculated from theformula:

$$TGI = [(Vcontrol - Vexperiment)/Vcontrol] \times 100\%;$$

mean lifetime (ML), lifetime increase (LI) calculated from the formula:

$$LI = [(MLexperiment - MLcontrol)/Mlcontrol] \times 100\%.$$

TGI > 50%, LI > 50% were considered to be biologically significant effects.

The obtained data were processed in accordance with the Fisher-Student procedure, using statistical programs. The differences were regarded confident at $p < 0.05$.

The results of action of the vegetable extracts are presented in Table 4, from which it is seen that preparations 2 and 3 are able to inhibit the growth of L1210 in vivo.

The results of action of the vegetable extracts on the curative effect of DDP are presented in Table 5. As can be seen from Table 5, preparations 2 and 3 increase the therapeutic effect of the cytostatic, bringing about an increase of the TGI value by the 6th, 8th days of the tumor growth.

Table 4

| Influence of vegetable extract samples on L-1210 growth | | | | | |
|---|---|---|---|---|---|
| Ex. No. | Dose, ml/kg | TGI, % days | | | LI, % |
| | | 4 | 6 | 8 | |
| 2 | 5.4 | 26 | 46 | 36 | 24 |
| 2d | 5.4 | 28 | 48 | 39 | 10 |
| 2f | 5.4 | 20 | 44 | 35 | 12 |
| 2 | 10.8 | 41 | 38 | 37 | 2 |
| 2d | 10.8 | 41 | 36 | 37 | 8 |
| 2f | 10.8 | 40 | 37 | 34 | 5 |
| 3 | 5.4 | 56 | 24 | 25 | -7 |
| 3c | 5.4 | 50 | 25 | 24 | 2 |
| 3f | 5.4 | 57 | 25 | 24 | -1 |
| 3 | 10.8 | 65 | 27 | 22 | -7 |
| 3c | 10.8 | 67 | 27 | 23 | 4 |
| 3f | 10.8 | 65 | 30 | 21 | 0 |

| Influence of vegetable extract samples on curative effect of DDP | | | | | |
|---|---|---|---|---|---|
| Action | Dose, ml/kg | TGI, % days | | | LI, % |
| | | 4 | 6 | 8 | |
| DDP | 9 mg/kg | 100 | 93 | 33 | 41 |
| DDP +2 | 10.8 | 100 | 92 | 43 | 37 |
| DDP +2d | 10.8 | 100 | 90 | 39 | 40 |
| DDP +2f | 10.8 | 100 | 90 | 38 | 35 |
| DDP +3 | 10.8 | 100 | 92 | 69 | 30 |
| DDP +2b | 5.4 | 100 | 40 | 47 | 11 |

(continued)

| Influence of vegetable extract samples on curative effect of DDP | | | | | |
|---|---|---|---|---|---|
| Action | Dose, ml/kg | TGI, % days | | | LI, % |
| | | 4 | 6 | 8 | |
| DDR +2e | 5.4 | 100 | 41 | 40 | 8 |
| DDP +3 | 5.4 | 100 | 63 | 41 | 23 |
| DDP +3c | 5.4 | 100 | 65 | 44 | 20 |
| DDP +3e | 5.4 | 100 | 66 | 44 | 24 |
| DDP | 10 mg/kg | 100 | 87 | 65 | 32 |
| DDP +2 | 5.4 | 100 | 100 | 88 | 33 |
| DDP +2c | 5.4 | 100 | 98 | 87 | 29 |
| DDP +2e | 5.4 | 100 | 100 | 90 | 32 |
| DDP +3 | 5.4 | 100 | 95 | 74 | 31 |
| DDP +3d | 5.4 | 100 | 91 | 71 | 32 |
| DDP +3e | 5.4 | 100 | 97 | 79 | 30 |

As can be seen from Tables 4 and 5, preparations 2 and 3 do not reduce the therapeutic effect of DDP, and under definite administration conditions they even enhance it. Preparation 3 has its own weakly pronounced antitumoric effect, causing a biologically significant inhibition of tumor growth in the early periods of the tumor development.
Hence, the vegetable extracts may be promising for use in oncological practice, when included into antitumoric therapy procedures.

**Claims**

1. A composition for treating oncological diseases in the form of an aqueous extract of vegetable stock, comprising extracts of plants of the genera Geranium, Plantago and a plant of the species Calendula officinalis as well as a plant of the species Pentaphylloides fruticosia, prepared with the following ratio of plants and water, g:

| | |
|---|---|
| Geranium | 10-60 dry or 300-360 fresh |
| Plantago | 10-60 dry |
| Calendula officinalis | 10-60 dry |
| Pentaphylloides fruticosa | 10-60 dry |
| Water | 3000 |

2. Composition according to Claim 1, **characterized in that** it additionally comprises a plant of the species Angelica, in an amount of 10-60 g dry.

3. A process for preparing an aqueous extract from vegetable stock for treating oncological diseases, **characterized in that** plants of the genera Geranium, Plantago and a plant of the species Calendula officinalis and a plant of the species Pentaphylloides fruticosa are macerated in water, this being followed by straining and centrifugation.

4. A process according to Claim 3, **characterized in that** the plant Angelica is additionally introduced into the composition of vegetable stock for maceration.

**Patentansprüche**

1. Zusammensetzung zur Behandlung von onkologischen Krankheiten in der Form eines wässerigen Extrakts von pflanzlichem Ausgangsmaterial, **dadurch gekennzeichnet, dass** sie Extrakte von Pflanzen der Gattungen Geranium, Plantago und einer Pflanze der Spezies Calendula officinalis sowie einer Pflanze der Spezies Pentaphylloides fruticosia umfasst, hergestellt mit dem nachfolgenden Anteil an Pflanzen und Wasser in g:

| Geranium | 10-60 trocken oder 300-360 frisch |
|---|---|
| Plantago | 10-60 trocken |
| Calendula officinalis | 10-60 trocken |
| Pentaphylloides fruticosa | 10-60 trocken |
| Wasser | 3000 |

**2.** Zusammensetzung gemäß Anspruch 1, **dadurch gekennzeichnet, dass** sie zusätzlich einen Extrakt einer Pflanze der Spezies Angelika umfasst, hergestellt aus 10-60 g der besagten trockenen Pflanze

**3.** Verfahren zur herstellung eines wässerigen Extrakts aus pflanzlichem Ausgangsmaterial zur Behandlung onkologischer Krankheiten, **dadurch gekennzeichnet, dass** Pflanzen der Gattungen Geranium, Plantago and eine Pflanze der Spezies Calendula officinalis and eine Pflanze der Spezies Pentaphylloides fruticosa in Wasser mazeriert werden, mit nachfolgendem Filtrieren und Zentrifugation.

**4.** Verfahren gemäß Anspruch 3, **dadurch gekennzeichnet, dass** die Pflanze Angelika zusätzlich in die Zusammensetzung des pflanzlichen Ausgangsmaterials zur Mazeration eingebracht wird.


**Revendications**

**1.** Une composition pour traiter des affections oncologiques sous la forme d'un extrait aqueux dune charge végétale, **caractérisée en ce qu'**elle comprend des extraits de plantes (du genre geranium, plantain, et une plante de l'espèce calendula officinalis ainsi qu'une plante de l'espèce pentaphylloides fruticosa), obtenue à partir du rapport suivant des plantes et de l'eau:

| Geranium | 10-60 à à sec ou 300-360 fraîche |
|---|---|
| Plantain | 10-60 à à sec |
| Calendula officinalis | 10-60 à à sec |
| Pentaphylloides fruticosa | 10-60 à à sec |
| Eau | 3000 |

**2.** Composition selon la revendication 1, **caractérisée en ce qu'**elle contient en outre un extrait dune plante de l'espèce angelica obtenue à partir de 10 à 60g de plante sèche.

**3.** Un procédé de préparation d'un extrait aqueux dune charge végétale pour le traitement d'affections oncologiques, **caractérisé en ce que** des plantes (du genre geranium, plantain, et une plante de l'espèce calendula officinalis ainsi qu'une plante de l'espèce pentaphylloides fruticosa), sont mis à macérer dans de l'eau, macération que l'on fait suivre d'un foulage puis d'une centrifugation.

**4.** Un procédé selon la revendication 3, **caractérisé en ce que** l'on introduit en outre, avant macération, une plante de l'espèce angelica dans la composition de ladite charge végétale.

FIG. 1